# EUROPÄISCHE PATENTSCHRIFT

(11) **EP 1 084 266 B1**
(45) Veröffentlichungstag und Bekanntmachung des Hinweises auf die Patenterteilung: **30.06.2004**
(21) Anmeldenummer: 99917961.7
(22) Anmeldetag: 06.04.1999
(51) Int. Cl.: C12Q 1/68, C12P 19/00

(54) **ANWENDUNG VON 5'-MODIFIZIERTEN NUKLEOTIDEN IN DER MOLEKULARBIOLOGIE UND MEDIZIN**
THE APPLICATION OF 5'-MODIFIED NUCLEOTIDES IN MOLECULAR BIOLOGY AND MEDICINE
UTILISATION DE NUCLEOTIDES 5'-MODIFIES EN BIOLOGIE MOLECULAIRE ET EN MEDECINE

(30) Priorität: 08.04.1998 DE 19815864
(43) Veröffentlichungstag der Anmeldung: 21.03.2001
(73) Patentinhaber: Europäisches Laboratorium für Molekularbiologie, D-69117 Heidelberg (DE)
(72) Erfinder: FAULSTICH, Konrad, D-69117 Heidelberg (DE); ANSORGE, Wilhelm, D-69117 Heidelberg (DE)
(74) Vertreter: Weiss, Wolfgang, Dipl.-Chem. Dr.
(86) Internationale Anmeldenummer: PCT/EP1999/002320
(87) Internationale Veröffentlichungsnummer: WO 1999/053087

(56) Entgegenhaltungen:
- WO-A-95/06752
- WO-A-96/19572
- DE-A- 3 446 635
- LETSINGER, ROBERT L.; WILKES, JOHN S.; DUMAS, LAWRENCE B.: "Nucleotide chemistry. XVII. Enzymic synthesis of polydeoxyribonucleotides possessing internucleotide phosphoramidate bonds" J. AMER. CHEM. SOC., Bd. 94, Nr. 1, 1972, Seiten 292-3, XP002112658
- CHEN, MING S.; WARD, DAVID C.; PRUSOFF, WILLIAM H.: "5-Iodo-5'-amino-2',5'-dideoxyuridine-5'-N '-triphosphate. Synthesis, chemical properties, and effect on Escherichia coli thymidine kinase activity" J. BIOL. CHEM., Bd. 251, Nr. 16, 1976, Seiten 4839-41, XP002112659
- MAG, MATTHIAS; ENGELS, JOACHIM W.: "Synthesis and selective cleavage of oligodeoxyribonucleotides containing non-chiral internucleotide phosphoramidate linkages" NUCLEIC ACIDS RES., Bd. 17, Nr. 15, 1989, Seiten 5973-88, XP002112660
- MAG, MATTHIAS; LUEKING, SILKE; ENGELS, JOACHIM W.: "Synthesis and selective cleavage of an oligodeoxynucleotide containing a bridged internucleotide 5'-phosphorothioate linkage" NUCLEIC ACIDS RES., Bd. 19, Nr. 7, 1991, Seiten 1437-41, XP002112661
- TROWBRIDGE, DALE B.; YAMAMOTO, DIANE M.; KENYON, GEORGE L.: "Ring openings of trimetaphosphoric acid and its bismethylene analog.Syntheses of adenosine 5'-bis(dihydroxyphosphinylmethyl) phosphinate and 5'-amino-5'-deoxyadensoine 5'-triphosphate" J. AMER. CHEM. SOC., Bd. 94, Nr. 11, 1972, Seiten 3816-24, XP002112662
- PATEL, BHISMA KUMAR; ECKSTEIN, FRITZ: "5'-Deoxy-5'-thioribonucleoside-5'-triphos phates" TETRAHEDRON LETT., Bd. 38, Nr. 6, 1997, Seiten 1021-4, XP002112663
- STUETZ, ANTON; SCHEIT, KARL H.: "Properties of ATP and UTP analogs with phosphorus-sulfur-carbon-5'-bonds" EUR. J. BIOCHEM., Bd. 50, Nr. 2, 1975, Seiten 343-9, XP002112664

## Beschreibung

Die Erfindung betrifft Verfahren zum Einbau von 5'-modifizierten Nukleotiden in Nukleinsäuren und die anschließende ortspezifische Spaltung der Nukleinsäuren an den 5'-modifizierten Monomerbausteinen. Diese Verfahren können zur Nukleinsäuresequenzierung, zur Erzeugung von Nukleinsäurebibliotheken oder zum Nachweis von Mutationen, eingesetzt werden.

Die heute routinemäßig angewandten Verfahren zur Sequenzierung von Nukleinsäuren beinhalten im allgemeinen die Polymerisation eines zu einer Matrize komplementären Nukleinsäurestrangs und die Erzeugung eines Gemisches an Nukleinsäurefragmenten mit allen möglichen Längen (1). Die Sequenzierung dieses Nukleinsäurefragmentgemisches kann durch Termination der Polymerisation oder Abbau durch Exonukleasen (2), iterative Sequenziermethoden (3), Zugabe einzelner Basen und Nachweis der Freisetzung von Pyrophosphat (4), chemische Methoden unter Verwendung von Eliminationsreaktionen (5), chemisch-enzymatische Methoden unter Einbau von modifizierten Nukleosiden und Spaltung durch Angriff auf Phosphorthioat- oder Bormodifizierte Nukleotide (6) bzw. auf 5'-amino-modifizierte Nukleotide (28,29), Einbau von Ribonukleosiden in DNA und anschließende Spaltung unter basischen Bedingungen (7) oder den Einbau von 3'-farbmarkierten Nukleotiden mit gleichzeitiger oder nachfolgender Abspaltung des Farbstoffs (8) erfolgen. Neben diesen Methoden stehen auch Strategien zur Verfügung, die eine Sequenzierung durch Hybridisierung (9) und eine physikalische Fragmenterzeugung durch Massenspektrometrie (10) beinhalten. Auch ein Nachweis durch Atomkraftmikroskopie (11) wurde diskutiert.

In den letzten zwanzig Jahren ist die Methode der Wahl jedoch die enzymatische Kettenabbruchmethode gewesen. Diese Methode ermöglicht eine Automatisierung und eine Sequenzierung mit hohem Durchsatz zur Anwendung bei der Sequenzierung ganzer Genome. Die Automatisierung wurde durch Verwendung von Farbstoffprimern (12), interne Markierung (13) oder Farbstoffterminatoren (14) erreicht. Die Sequenzierung mit Farbstoffprimern und die interne Markierung haben jedoch den Nachteil, daß irreguläre Terminationsvorgänge in der Sequenzleiter auftreten und zur Fehlinterpretation der Sequenzdaten führen können. Farbstoffterminatoren haben den Nachteil, daß sie zum Teil an falschen Stellen eingebaut werden und nur eine begrenzte Ableselänge ermöglichen, da es sich bei ihnen um modifizierte Substrate handelt.

Darüber hinaus besteht ein Bedürfnis, die für eine Sequenzbestimmung benötigte DNA-Menge zu verringern. Hierzu steht derzeit nur eine einzige Zyklussequenzierungsmethode zur Verfügung (15), die jedoch im Gegensatz zu PCR, wo eine exponentielle Amplifikation stattfindet, nur zu einer linearen Amplifikation der Produkte führt. Der Einsatz yon PCR-Amplifikation der Probe ist in einem Sequenzierungsverfahren möglich, das unter Verwendung Boranmodifizierter Nukleinsäuren einen enzymatischen Nukleinsäureaufbau und anschließenden gezielten Abbau vorsieht (30). Die direkte Sequenzierung von PCR-Produkten weist wiederum Nachteile auf, da in den Reaktionsgefäßen größere Mengen an Triphosphaten und Primermolekülen vorliegen, die zu einer Beeinträchtigung der Sequenzierungsreaktion oder der Sequenzbestimmung führen können (16). Die Aufreinigung von PCR-Produkten ist jedoch zeitaufwendig und bedeutet einen zusätzlichen Verfahrensschritt. Zwar können Triphosphate durch enzymatische Methoden (17) gespalten werden, aber auch dies ist zeitaufwending und erhöht die Kosten für die Durchführung der Sequenzierungsreaktion. Als Alternative steht ein direktes exponentielles Amplifikations- und Sequenzierverfahren (DEXAS) zur Sequenzierung geringer Mengen an DNA-Material zur Verfügung (18); dieses Verfahren konnte bisher jedoch nicht für eine Standardsequenzierung eingesetzt werden und ist - im Gegensatz zu seinem Namen - nicht direkt exponentiell.

Gemäß vorliegender Erfindung wird ein neues Nukleinsäuresequenzierungsverfahren bereitgestellt, bei dem die Nachteile des Standes der Technik mindestens teilweise vermieden werden. Durch dieses Verfahren wird insbesondere das Problem der Substratspezifität hinsichtlich Farbstoffterminatoren vermieden und es ermöglicht eine schnelle DNA-Sequenzierung unter Verwendung sehr geringer Mengen an DNA-Ausgangsmaterial in Kombination mit einer Nukleinsäureamplifikationsreaktion wie etwa PCR. Auch die lesbare Länge der sequenzierbaren Matrizen wird durch das Verfahren verbessert.

Das erfindungsgemäße Verfahren zum Nachweis von Nukleinsäuren umfasst das Einbauen von Verbindungen der allgemeinen Formel (I): 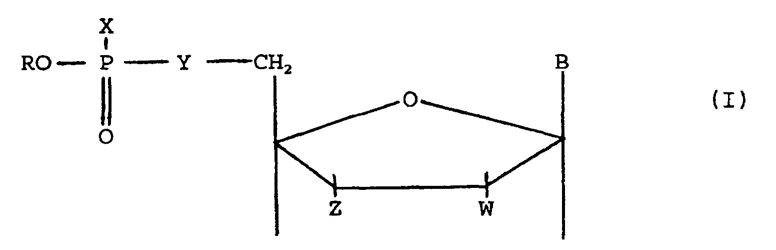 worin:
- B: eine Nukleobase, d.h. eine natürliche oder nichtnatürliche zur Hybridisierung mit komplementären Nukleinsäuresträngen geeignete Base wie etwa A, C, G, T, U, I, 7-Deaza-G, 7-Deaza-A, 5-Methyl-C etc. bedeutet,
- W und Z: jeweils OR¹, SR¹, N(R¹)₂ oder R¹ bedeuten, wobei R¹ jeweils unabhängig bei jedem Vorkommen Wasserstoff oder einen organischen Rest, z.B. einen Alkyl-, Alkenyl-, Hydroxyalkyl-, Amin-, Ester-, Acetal- oder Thioesterrest, vorzugsweise mit bis zu 10 Kohlenstoffatomen und besonders bevorzugt mit bis zu 6 Kohlenstoffatomen darstellt,
- X: OR², SR² oder B(R²)₃ bedeutet, wobei R² jeweils unabhängig Wasserstoff, ein Kation, z.B. ein Alkalimetall- oder Ammoniumion, oder einen organischen Rest, z.B. einen Farbstoff wie etwa Fluoresceine, Rhodamine, Cyanine und deren Derivate bedeutet,
- Y: NR³ oder S, insbesondere NR³ bedeutet, wobei R³ Wasserstoff oder einen organischen Rest, z.B. einen gesättigten oder ungesättigten Kohlenwasserstoffrest, insbesondere einen C₁-C₄-Rest oder einen Farbstoffrest darstellt, wobei unter Wasserstoff auch die Isotopen Deuterium und Tritium zu verstehen sind, und
- R: Wasserstoff, ein Kation, einen organischen Rest oder eine gegebenenfalls modifizierte Phosphat- oder Diphosphatgruppe, insbesondere eine Diphosphatgruppe bedeutet,
in Nukleinsäuren und die anschließende ortsspezifische Spaltung der Nukleinsäuren, vorzugsweise durch Hydrolyse der Bindung P-Y, wobei Nukleinsäurefragmente mit einem 5'-Ende HY-CH₂- entstehen.

Die Gruppe R kann einen organischen Rest bedeuten, bespielsweise einen lipophilen Rest, der das Eindringen der Substanz in eine Zelle erleichtert. Vorzugsweise ist R eine Phosphatgruppe: 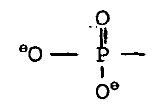 oder eine Diphosphatgruppe:

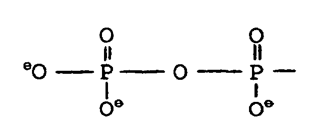

Diese Phosphat- oder Diphosphatgruppe kann modifiziert sein. So können ein oder mehrere endständige Sauerstoffatome Substituenten tragen, z.B. organische Reste. Andererseits können ein oder mehrere endständige Sauerstoffatome und - bei der Diphosphatgruppe - auch das Brückensauerstoffatom durch Gruppen wie S, NR³ oder C(R³)₂ ausgetauscht sein, wobei R³ wie zuvor definiert ist. Darüber hinaus können auch 2 Substituenten an endständigen Sauerstoffatomen miteinander verbrückt sein.

Wenn Substituenten vorhanden sind, befinden sie sich vorzugsweise an Sauerstoffatomen des jeweils endständigen Phosphoratoms, besonders bevorzugt am γ-Phosphoratom. Beispiele für geeignete Substituenten sind organische Reste wie etwa Alkylreste, die selbst substituiert sein können, oder eine Salicylgruppe, die mit 2 Sauerstoffatomen des endständigen Phosphors einen 6-gliedrigen cyclischen Diester bilden kann. Der aromatische Kern der Salicylgruppen kann wiederum selbst einen oder mehrere zusätzliche Substituenten tragen, z.B. solche wie für R¹ definiert oder Halogenatome. Weiterhin bevorzugte Substituenten am Sauerstoffatom sind Reste wie C₁-C₁₀-Alkyl, -(CH₂)ₙ-N₃, (CH₂)ₙN(R³)₂ oder -(CH₂)ₙNHOCO(CH₂)ₘ-N(R³)₂, wobei n und m ganze Zahlen von 1 bis 8, vorzugsweise von 2 bis 5 sind und R³ wie zuvor definiert ist, aber außerdem vorzugsweise einen aromatischen Rest wie etwa Phenyl- oder Dinitrophenyl bedeuten kann.

Der Einbau von Verbindungen der allgemeinen Formel (I) in Nukleinsäuren erfolgt vorzugsweise enzymatisch. Möglich ist jedoch auch eine chemische Synthese. Für einen enzymatischen Einbau werden vorzugsweise Enzyme verwendet ausgewählt aus der Gruppe bestehend aus DNA-abhängigen DNA-Polymerasen, DNA-abhängigen RNA Polymerasen, RNA-abhängigen DNA Polymerasen, RNA-abhängigen RNA Polymerasen und Terminalen Transferasen. Besonders bevorzugt ist die T7 DNA-Polymerase, verwandte Enzyme wie etwa die T3 oder die SP6 DNA Polymerase oder Modifikationen dieser Enzyme. Dementsprechend kann es sich bei den Nukleinsäuren, in die die Verbindungen der Formel (I) eingebaut werden, um DNAs oder/und RNAs handeln, die gegebenenfalls ein oder mehrere weitere modifizierte Nukleotidbausteine tragen können.

Nukleinsäuren, die als Monomerbausteine mindestens eine Verbindung der allgemeinen Formel (I) enthalten, können an dem die P-Y Bindung enthaltenden Nukteotidbaustein ortsspezifisch gespalten werden. Diese ortsspezifische Spaltung kann beispielsweise an der P-Y-Bindung selbst durch Temperaturerhöhung, z.B. auf mindestens 37°C, Einstellung saurer Bedingungen, z.B. pH ≤ 5, Mikrowellenbehandlung, Laserbehandlung, z.B. mit einem Infrarotlaser oder/und 3'-seitig des die P-Y-Bindung enthaltenden Nukleotids durch enzymatischen Verdau, beispielsweise mit Exo- oder Endonukleasen oder Phosphodiesterasen, z.B. 3'→5'-Schlangengiftphosphodiesterase erfolgen.

Das erfindungsgemäße Verfahren kann auch in Kombination mit einer Amplifikationsreaktion, z.B. einer PCR, durchgeführt werden. Dies erlaubt die Verwendung von extrem geringen Mengen an DNA-Ausgangsmaterial zur Erzeugung markierter komplementärer Nukleinsäurestränge. Vorzugsweise wird die Nukleinsäureamplifikation mit thermostabilen Enzymen in mehreren Zyklen durchgeführt.

Der Einbau der Verbindungen gemäß (I) in die Nukleinsäuren kann in Lösung erfolgen. Alternativ kann der Einbau der Verbindungen jedoch auch in trägergebundene Nukleinsäuren erfolgen. Nach der Synthese kann dann eine Freisetzung der Nukleinsäuren vom Träger, gegebenenfalls durch die ortsspezifische Spaltung der P-Y-Bindung oder durch andere Methoden erfolgen.

Nach der ortsspezifischen Spaltung der Nukleinsäuren werden Nukleinsäurefragmente erzeugt, die vorzugsweise an ihrem 5'-Ende die Gruppe Y-CH₂oder/und an ihrem 3'-Ende eine Phosphatgruppe aufweisen. Bisher mußten derart modifizierte Nukleinsäuren auf komplizierte Art und Weise durch chemische Synthese (19) der durch enzymatische Reaktionen (20, 21) erzeugt werden. Das erfindungsgemäße Verfahren ist wesentlich schneller, kostengünstiger und erlaubt eine einfachere Handhabung der Verbindungen. Die auf diese Weise hergestellten modifizierten Nukleinsäuren können für therapeutische Zwecke oder/und für molekularbiotogische Untersuchungen, z.B. Untersuchungen von Mechanismen der Aufnahme und des Metabolismus von Nukleinsäuren in Zellen verwendet werden, da an die 5'-Y-Gruppe auf einfache Weise eine Markierungsgruppe gekoppelt werden kann. Die 3'-Phosphatgruppe wiederum stellt eine Schutzgruppe gegenüber einer Ligation oder/und einer enzymatischen Elongation mit Polymerasen dar. An das phosphorylierte 3'-Ende der Nukleinsäurefragmente können - sofern gewünscht - Markierungsgruppen angefügt werden, z.B. wenn eine Dephosphorylierung durchgeführt wird und an die resultierende 3'-OH-Gruppe durch eine enzymatische Reaktion beispielsweise unter Verwendung von Ligase oder Terminaler Transferase markierte Oligonukleotide oder mit einer Polymerase markierte Dideoxynukleosidtriphosphate angefügt werden oder wenn die 3'-Phosphatgruppe eine reaktive Gruppe z.B. ein Schwefelatom enthält.

Die durch das erfindungsgemäße Verfahren erzeugten Nukleinsäurefragmente können weiterhin aufgrund
der definierten Gruppe an ihrem 5'-Ende auf einfache Weise auf einem Träger immobilisiert werden, der eine mit der Gruppe Y reaktive, funktionalisierte Oberfläche enthält. Andererseits kann auch eine adsorptive Bindung über die Gruppe Y an eine Oberfläche erfolgen. Geeignete Träger sind solche, die Oberflächen beispielsweiseaus Metall, Glas, Keramiken oder/und Kunststoff aufweisen. Besonders bevorzugt sind Träger mit Glas oder/und Siliciumoberflächen. Die Träger können weiterhin jede beliebige Form aufweisen, z.B. Mikropartikel wie etwa magnetische Mikropartikel oder Halbleitermaterialien wie Biochips, z.B. DNA- oder RNA-Chips, die gegebenenfalls mehrere mit Nukleinsäuren spezifisch bindefähige definierte Flächen in Form von Array-Anordnungen enthalten können.

Die durch das erfindungsgemäße Verfahren erzeugten Nukleinsäurefragmente können auch in Form eines
Gemisches unterschiedlicher Fragmente an einen Träger gekoppelt werden. Auf diese Weise werden Träger mit statistisch darauf angeordneten Nukleinsäurefragmenten erzeugt. Dies hat Vorteile, wenn z.B. eine anschließende Amplifikation auf der Trägeroberfläche mit Primern durchführt, die für eine vorbestimmte Nukleinsäuresequenz, z.B. ein Gen kodieren.

Wenn bei der Spaltung der Nukleinsäuren ein heterogenes Nukleinsäuregemisch entsteht, kann dieses zur Herstellung einer Nukleinsäurebibliothek, insbesondere einer statistischen Bibliothek verwendet werden. Solche Bibliotheken können auch durch multiplen statistischen Einbau von Verbindungen der Formel (I) in einen Nukleinsäurestrang gefolgt von einer ortsspezifischen Spaltung erzeugt werden. Außerdem können zur Erzeugung statistischer Nukleinsäurebibliotheken auch degenerierte und statistisch an Nukleinsäurematrizen bindende Primer eingesetzt werden.

Die Fragmente der Nukleinsäurebibliothek können kombinatorisch ohne oder nach weiterer enzymatischer oder chemischer Behandlung wieder zusammengesetzt werden (DNA-Shuffling). Da bei jedem Fragment das 5'-Ende mit einer Gruppe Y versehen ist (mit Ausnahme des 5'-Endes des ersten Fragments), kann das Zusammensetzen anderer Fragmente nur so erfolgen, daß das ursprüngliche erste Fragment wiederum das erste Fragment bildet. Den vollständigen kombinatorischen Raum kann man nach weiterer enzymatischer oder chemischer Behandlung der Bibliothek oder einzelne Fragmente daraus ausnutzen.

Die durch das erfindungsgemäße Verfahren erzeugten Nukleinsäurefragmente können nach der ortsspezifischen Spaltung einer Nachweisreaktion unterzogen werden. Diese Nachweisreaktion kann mit beliebigen, für diesen Zweck bekannten Methoden erfolgen. Vorzugsweise wird eine massenspektrometrische Analyse oder/und eine Elektrophorese, z.B. eine Polyacrylamidgelelektrophorese, durchgeführt.

Die Nachweisreaktion kann beispielsweise zum Nachweis von Mutationen, z.B. von Punktmutationen in Nukleinsäuren eingesetzt werden. Zwei Protokolle zur Analyse von Punktmutationen werden im folgenden ausführlich beschrieben.

Eine weitere wichtige Anwendung des erfindungsgemäßen Verfahrens ist die Nukleinsäuresequenzierung. Solche Sequenzierverfahren können in unterschiedlichen Varianten durchgeführt werden. Beispielsweise ist das erfindungsgemäße Verfahren auch für eine "Cycle"-Sequenzierung in Kombination mit einer Nukleinsäureamplifikation oder/und für eine bidirektionale Sequenzanalyse auf einem Nukleinsäurestrang geeignet. Bevorzugte Beispiele von Sequenzierverfahren werden im folgenden ausführlich beschrieben.

Verbindungen der Formel (I) können als Bestandteile von Reagenzienkits zum Nachweis von Nukleinsäuren, z.B. als Sequenzierungskits oder als Kits zur Mutationsanalyse gegebenenfalls mit weiteren Nachweiskomponenten verwendet werden. Diese weiteren Nachweiskomponenten sind beispielsweise Enzyme, insbesondere Polymerasen wie etwa DNA-Potymerasen oder Reverse Transkriptasen, als Primer verwendbare Oligonukleotide, die gegebenenfalls an ihrem 5'-Ende oder/und an ihrer Seitenkette eine Markierung tragen können, Deoxynukleosidtriphosphate, die gegebenenfalls eine Markierung tragen können, Dideoxynukleosidtriphosphate (Kettenabbruchmoleküle), die gegebenenfalls eine Markierung tragen können, sowie weitere Reagenzien, z.B. Puffer etc. und feste Träger. Vorzugsweise enthalten die Reagenzienkits die in den nachfolgenden Figuren angegebenen Bestandteile

Weiterhin wird die Erfindung durch die nachfolgenden Figuren und Beispiele erläutert:

Es zeigen:
- Fig. 1: die schematische Darstellung der Synthese 5'-Amino-modifizierter Nukleoside;
- Fig. 2: die schematische Darstellung der Herstellung von 5'-Amino-modifizierten Nukleosidtriphosphaten;
- Fig. 3: die schematische Darstellung der Erzeugung 5'-Amino-modifizierter oder/und 3'-phosphorylierter DNA-Fragmente durch ortsspezifische Spaltung der P-N-Bindung bei eingebauten 5'-Aminonukleosidtriphosphaten;
- Fig. 4: die schematische Darstellung einer selektiven 5'- oder 3'-Markierung von Nukleinsäurefragmenten;
- Fig. 5: die schematische Darstellung der Abspaltung von Nukleinsäuren von festen Trägern;
- Fig. 6: die schematische Darstellung eines Sequenzierprotokolls unter Verwendung eines 5'-Farbstoff-markierten Sequenzierprimers;
- Fig. 7: ein alternatives Verfahren zur Erzeugung sequenzierbarer Fragmente durch Exonukleaseverdauung;
- Fig. 8: die schematische Darstellung einer elektrophoresefreien iterativen Sequenziermethode;
- Fig. 9: die schematische Darstellung einer bidirektionalen Sequenziermethode an einem einzigen Nukleinsäurestrang;
- Fig. 10: die Markierung der Nukleinsäurefragmente nach der Sequenzierreaktion durch Terminale Transferase;
- Fig. 11: eine erste Ausführungsform zum Nachweis von Punktmutationen;
- Fig. 12: eine zweite Ausführungsform zum Nachweis von Punktmutationen und
- Fig. 13: die Erzeugung einer DNA Bibliothek.

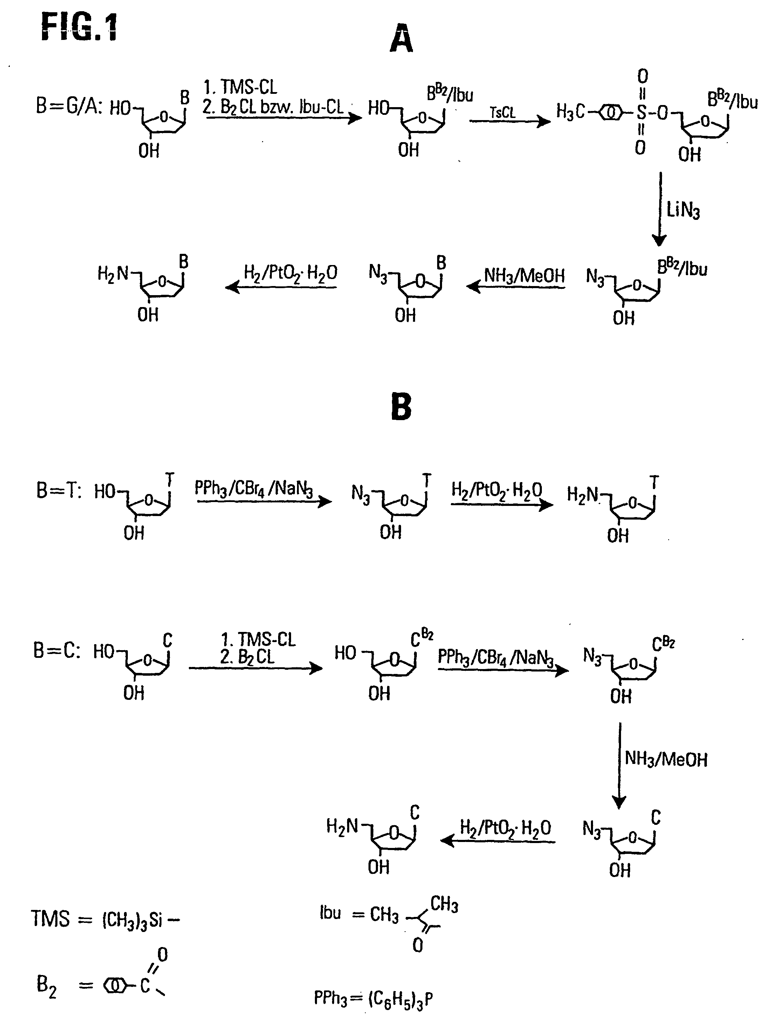

Verfahren zur Herstellung von Verbindungen der Formel (I), worin Y eine Aminogruppe darstellt, sind in Fig. 1a und b gezeigt. Fig. 1a zeigt ein Schema zur Herstellung von 5'-Amino-2',5'-dideoxypurinnukleosiden. Hierzu werden die Aminogruppen der Nukleobase durch Reaktion mit Schutzgruppen blockiert, z.B. durch Silylierung mit Trimethylsilylchlorid und anschließende Einführung einer Bz- oder Ibu-Schutzgruppe. Dann wird die 5'-OH-Gruppe aktiviert, z.B. durch Umsetzung mit Tosylchlorid, so daß sie mit einem Alkalimetallazid, z.B. LiN₃, reagieren kann. Nach Abspaltung der Schutzgruppen, z.B. durch NH₃/MeOH, kann die Schutzgruppe reduktiv, z.B. mit H₂/PtO₂, in eine Aminogruppe überführt werden.

In Fig. 1 b ist ein entsprechendes Syntheseschema zur Herstellung von 5'-Amino-2',5'-dideoxypyrimidinnukleosiden dargestellt. Thymidin kann beispielsweise direkt mit einem Azidsalz, z.B. NaN₃ umgesetzt und anschließend die Azidgruppe reduktiv in eine Aminogruppe überführt werden. Bei Cytidin wird zunächst die Nukleobase durch eine Schutzgruppe, z.B. Bz, blockiert und anschließend kann auf analoge Weise wie bei Thymidin eine Azidgruppe eingeführt werden, die reduktiv zu einer Aminogruppe umgesetzt werden kann.

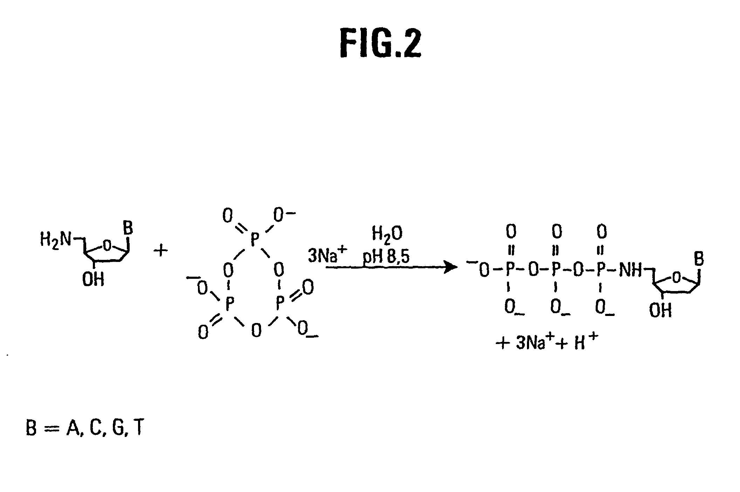

In Fig. 2 ist ein Syntheseschema zur Herstellung von 5'-Amino-2',5'dideoxynukleosid-5'-triphosphaten gezeigt, mit dem auf einfache Weise eine Triphosphatgruppe an die gemäß Fig. 1 erhaltenen Nukleoside angefügt werden kann. Die auf diese Weise hergestellten 5'-Aminonukleosidtriphosphate können als Monomerbausteine für den Einbau in Nukleinsäuren verwendet werden.

Ausführliche Angaben zur Ausführung dieser Reaktionen finden sich in Beispiel 1.

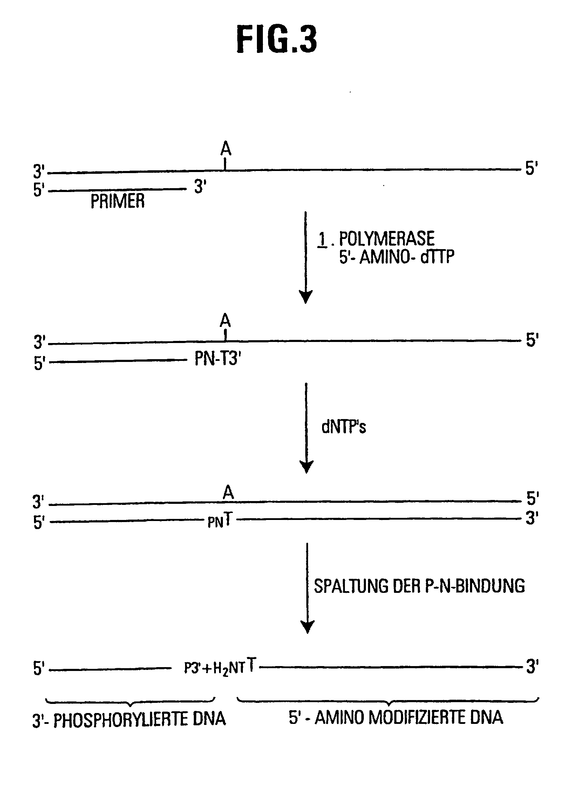

Fig. 3 zeigt die Erzeugung von modifizierten DNA-Fragmenten, die einen 5'-Amino-T-Baustein enthalten und die anschließende Spaltung dieser DNA-Fragmente an der P-N-Bindung, wobei 3'-phosphorylierte oder/und 5'-Amino-modifizierte DNA-Fragmente erhalten werden.

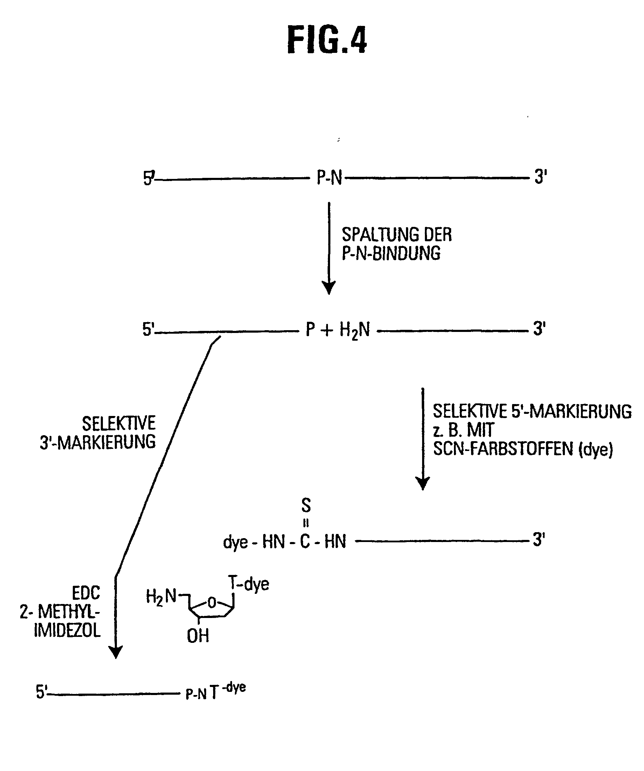

Fig. 4 zeigt Beispiele für eine selektive 5'- und 3'-Markierung der durch die Spaltung erzeugten Nukleinsäurefragmente.

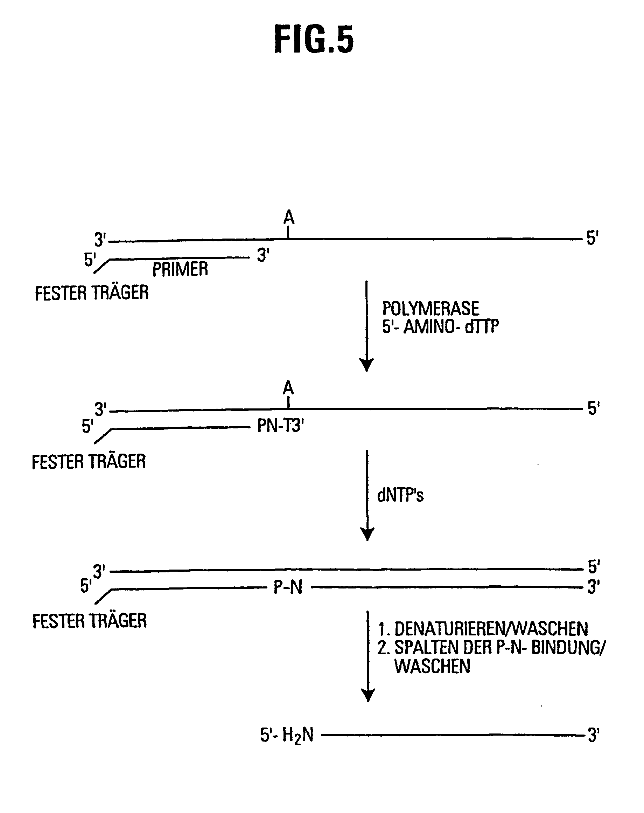

Fig. 5 zeigt die Synthese von 5'-Aminonukleotidbausteinen enthaltenen DNA-Molekülen an einen festen Träger und die anschließende Freisetzung 5'-Amino-modifizierter DNA-Fragmente durch Spaltung der P-N-Bindung.

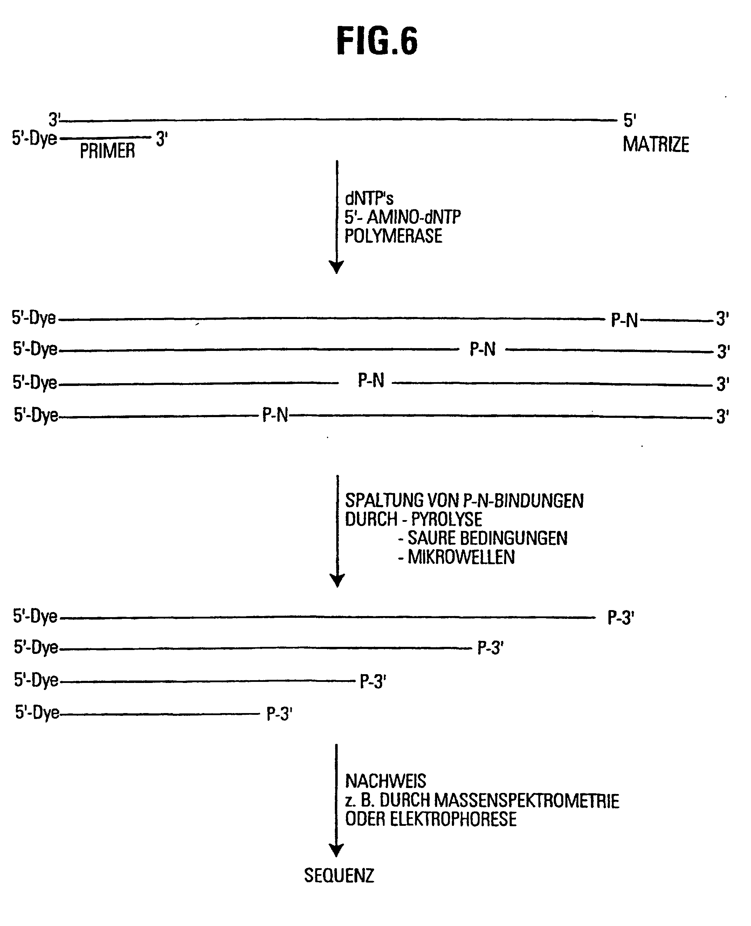

Fig. 6 zeigt eine schematische Darstellung einer Ausführungsform des 5'-Aminosequenzierverfahrens. Gemäß der gezeigten Ausführungsform wird ein am 5'-Ende eine Markierungsgruppe tragender Primer verwendet, der durch enzymatische Polymerisation verlängert wird, wobei 5'-Amino-modifizierte Nukleotide an statistischen Positionen in den Nukleinsäurestrang eingebaut werden. Die modifizierten Nukleotide werden von DNA Polymerasen, z.B. von der T7 DNA Polymerase, als Substrate akzeptiert. Die P-N-Bindungen sind statistisch über den Nukleinsäurestrang verteilt und können auf einfache Weise, z.B. durch Pyrolyse, saure Bedingungen oder/und Mikrowellenbehandlung gespalten werden, wobei ein Gemisch an Nukleinsäurefragmenten entsteht. Jedes dieser Fragmente trägt an seinem 3'-Ende eine Phosphatgruppe, wodurch Mobilitätsänderungen bei einer Gelelektrophorese vermieden werden. Bei Einbau von Verbindungen der Formel (I), bei denen X eine nachweisbare Gruppe, z.B. eine Farbstoffgruppe bedeutet, werden nach Spaltung Nukleinsäurefragmente erhalten, die an ihrem 3'Nukleotid eine Markierung aufweisen.

Auch für eine "Cycle"-Sequenzierung können die modifizierten Nukleotide eingesetzt werden. Unter Verwendung thermostabiler Polymerasen ist es möglich, die DNA-Matrize beispielsweise durch PCR zunächst zu amplifizieren und dann die modifizierten Nukleotide bei 37°C mit T7 DNA Polymerase einzubauen. Die Spaltung erfolgt dann direkt im Reaktionsgefäß durch einfaches Erhitzen auf beispielsweise 95°C. Alternativ kann der Einbau der modifizierten Nukleotide schon während der Amplifikation selbst durch eine thermostabile Polymerase erfolgen.

Nach der Spaltung wird das Reaktionsgemisch oder ein Teil davon einer Nachweisreaktion, z.B. durch Gelelektrophorese, unterzogen. Auf diese Weise werden Probleme hinsichtlich der Substratspezifität bei Farbstoffmarkierten Kettenabbruchmolekülen vermieden. Verglichen mit bisher verfügbaren chemisch-enzymatischen Methoden, z.B. durch Einbau von α-Thionukleotiden, erlaubt das erfindungsgemäße Verfahren eine einfachere Spaltung der erzeugten Nukleinsäurestränge, ohne daß aggressive Chemikalien verwendet werden müssen, die die Markierungsgruppe des Primers bzw. die glykosidische Bindung angreifen könnten. Durch das erfindungsgemäße Verfahren können die Kosten von bestehenden Sequenzierprotokollen verringert werden, da die Triphosphate auf sehr einfache Weise, z.B durch den Anwender selbst, direkt vor der Sequenzierung herstellbar sind. Das Verfahren ist schnell, einfach und funktioniert selbst mit einer sehr geringen Menge an DNA-Ausgangsmaterial.

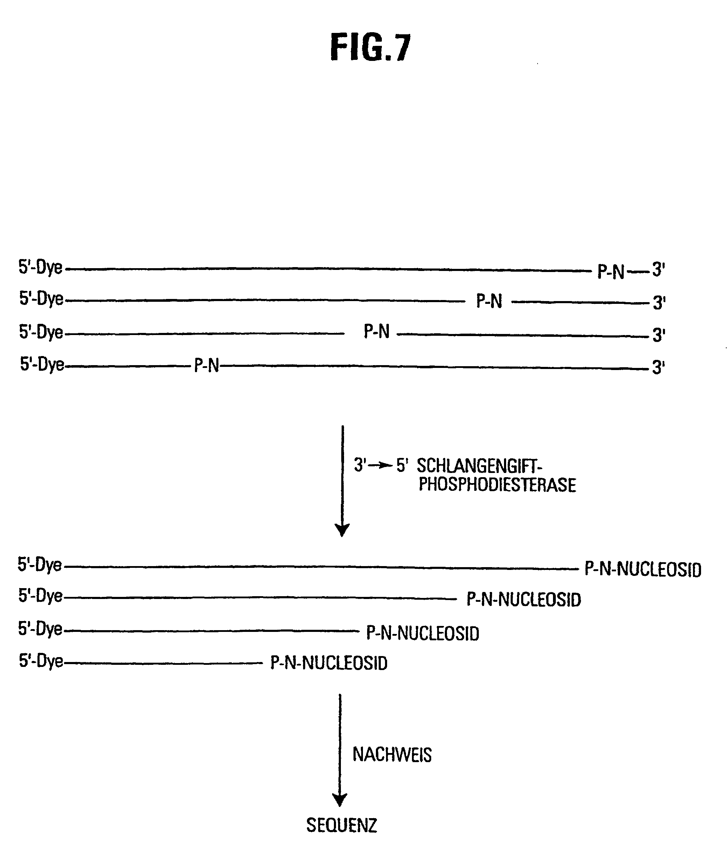

Fig. 7 zeigt eine alternative Methode zur Erzeugung von sequenzierbaren Nukleinsäurefragmenten durch 3'→5'-Exonukleaseverdau, z.B. durch Schlangengiftphosphodiesterase.

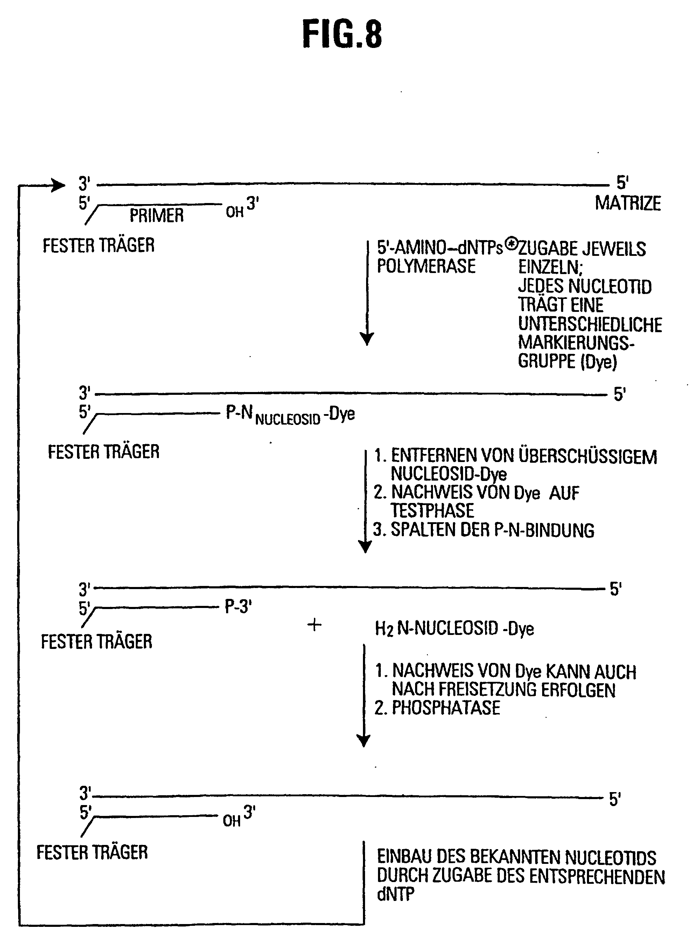

Fig. 8 zeigt ein Beispiel für eine Elektrophose- oder/und Gel-freie iterative Sequenziermethode. Dabei werden farbstoffmarkierte 5'-Amino-modifizierte Deoxy-nukleosidtriphosphate verwendet, wobei jedes Nukleotid eine unterschiedliche Markierungsgruppe trägt. Durch die DNA Polymerase wird ein einziges Farbstoff-markiertes Amino-modifiziertes Nukleosid an den Primer angefügt und anschließend spezifisch an der P-N-Bindung wieder abgespalten. Die Art des angelagerten Nukleotids kann durch die nachgewiesene Markierungsgruppe identifiziert werden. Anschließend kann das identifizierte Nukleotid in unmodifizierter Form durch die Polymerase angefügt und der zuvor beschriebene Sequenzierschritt wiederholt werden. Ein Mehrfacheinbau desselben Nukleotids kann durch die Intensität der Markierung, z.B. einer Fluoreszenzmarkierung, nachgewiesen werden.

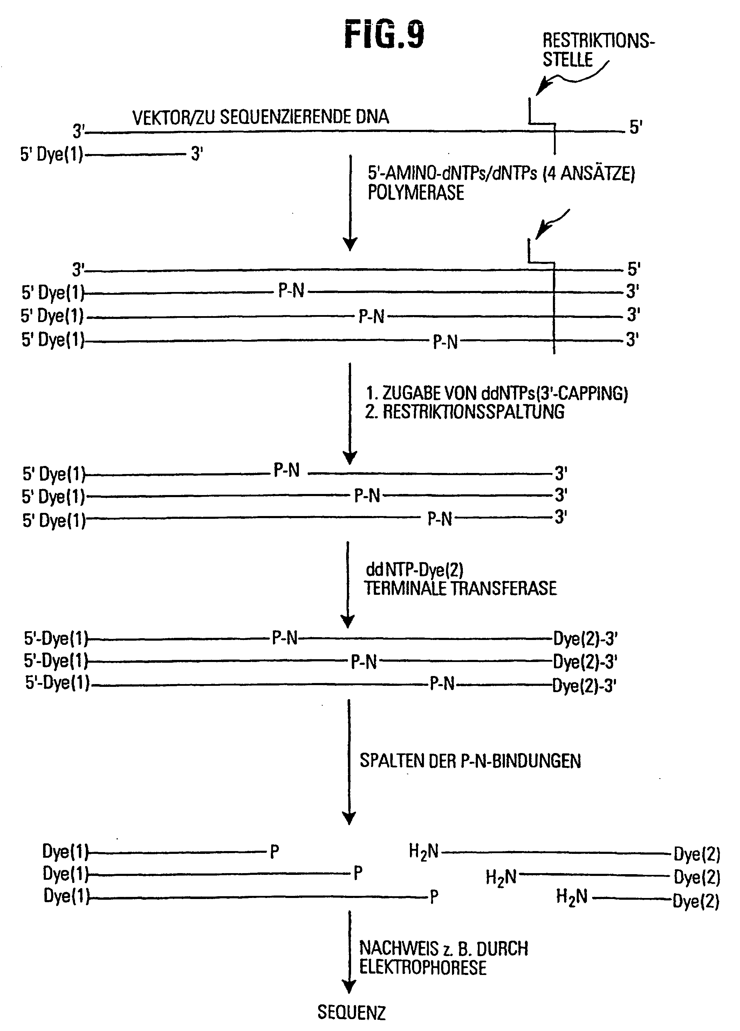

Fig. 9 zeigt ein bidirektionales Sequenzierprotokoll innerhalb eines einzigen Nukleinsäurestrangs. Hierzu werden wie zuvor beschrieben 5'-Aminomarkierte Nukleoside in Nukleinsäurestränge eingebaut. Die Termination der Elongation erfolgt durch Zugabe von Kettenabbruchmolekülen, z.B. ddNTPs. Durch Restriktionsspaltungen können dann definierte 3'-Enden der Nukleinsäurestränge erzeugt werden. Durch Zugabe eines mit einer zweiten Markierungsgruppe versehenen Kettenabbruchmoleküls, z.B. eines ddNTPs, und Terminaler Transferase werden Nukleinsäurestränge erhalten, die an ihrem 5'- bzw. 3'-Ende jeweils zwei, vorzugsweise unterschiedliche Markierungsgruppen tragen. Nach Spaltung der P-N-Bindungen werden zwei Sätze von unterschiedlich markierten DNA-Fragmenten erhalten, die in einer einzigen Sequenzreaktion nebeneinander nachweisbar sind.

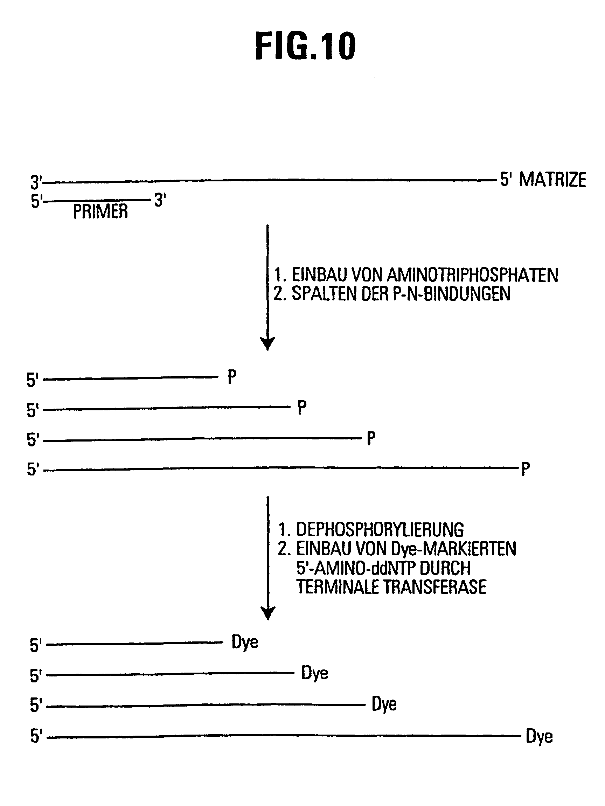

Fig. 10 zeigt eine 3'-terminale Einführung von Markierungsgruppen. Hierzu werden zunächst durch Polymerisation unter Verwendung von Aminotriphosphaten Nukleinsäurestränge hergestellt, die anschließend an der P-N-Bindung gespalten werden. Die resultierenden Nukleinsäurefragmente mit 3'-Phosphatgruppen werden dephosphoryliert und durch Zusatz eines eine Markierungsgruppetragenden Kettenabbruchmoleküls, z.B. eines 5'-AminoddNTPs, und Terminaler Transferase markiert. Auf diese Weise kann eine Sequenzierungreaktion in Abwesenheit jeglicher Art von Markierungsgruppen durchgeführt werden. Der Einbau der Markierungsgruppen in die zu sequenzierenden DNA Fragmente erfolgt erst nach Abschluß der Sequenzierungsreaktion. So werden Probleme hinsichtlich der Substratspezifität von Polymerasen vermieden und eine Verringerung der Kosten erreicht, da die Verwendung markierter Primermoleküle überflüssig wird.

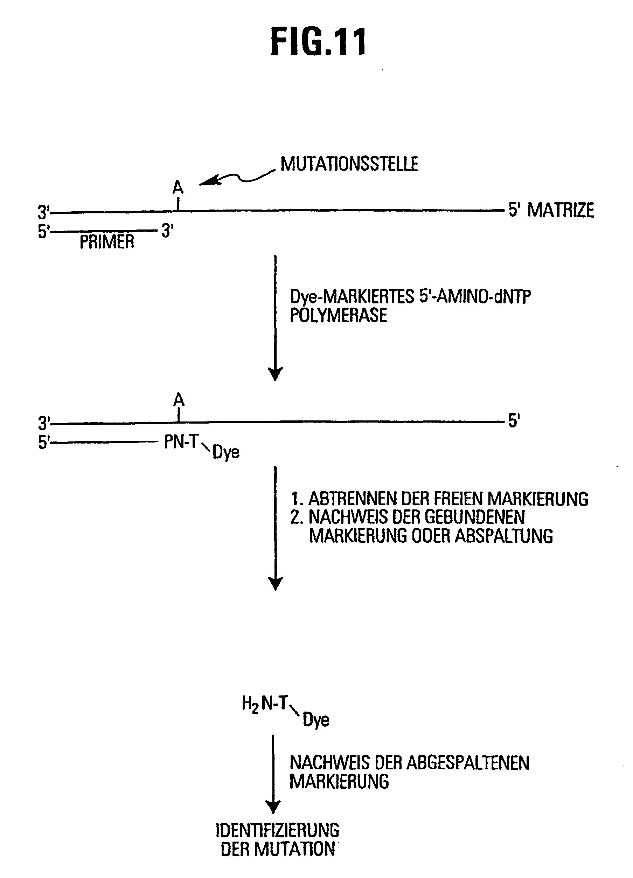

Fig. 11 zeigt eine erste Ausführungsform zum Nachweis von Punktmutationen. Dabei wird ein Nukleinsäureprimer verwendet, dessen 3'-Ende sich unmittelbar "stromaufwärts" der potentiellen Mutationsstelle befindet. Durch Zugabe eines mit einer Markierungsgruppe versehenen 5'-Amino-modifizierten Nukleosidtriphosphats in Gegenwart einer Polymerase erfolgt eine Elongation des Primers um mindestens ein Nukleotid,sofern auf dem Matrizenstrang das zu dem jeweils verwendeten modifizierten Nukleosidtriphosphat komplementäre Nukleotid vorliegt. Der Einbau des Aminomodifizierten markierten Nukleotids in den DNA-Strang oder das Ausbleiben dieses Einbaus läßt sich auf einfache Weise durch bekannte Methoden nachweisen. Hierzu kann das nichteingebaute Nukleotid z.B. durch Zentrifugation oder - bei Bindung an eine Festphase - durch Waschen entfernt und dann der Nachweis der Markierung in an den Primer gebundener Form oder/und nach Abspaltung erfolgen.

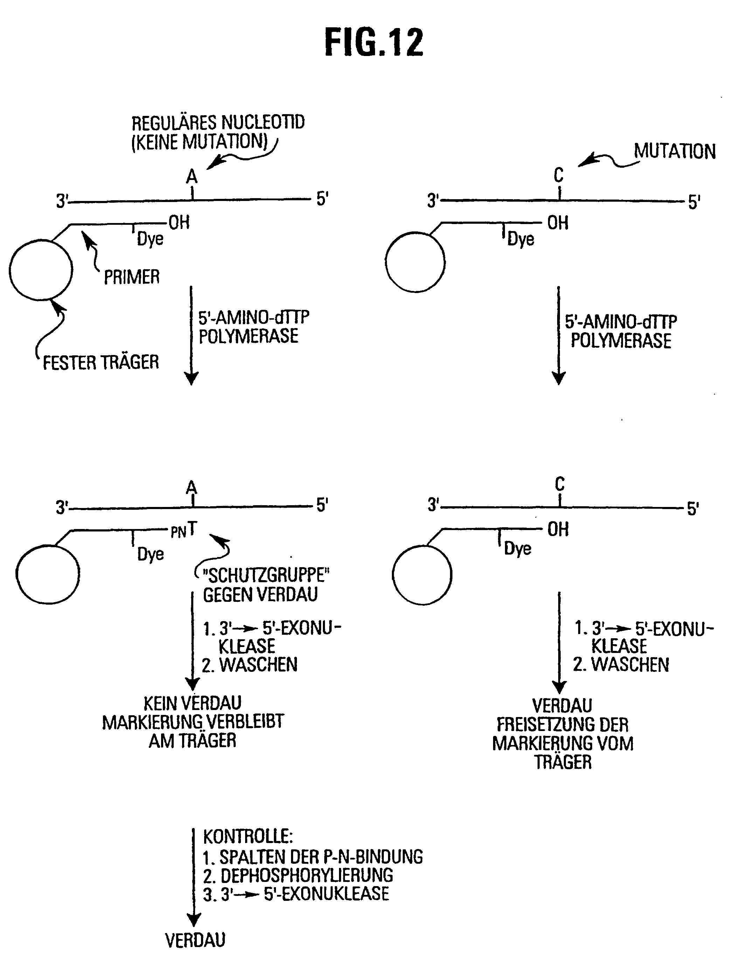

Fig. 12 zeigt eine weitere Ausführungsform zum Nachweis von Punktmutationen. Hierzu wird ein trägergebundener Primer verwendet, der eine interne Markierungsgruppe trägt. Dann werden 5'-Amino-modifiziertes Nukleosidtriphosphat und Polymerase zugegeben. Bei Vorliegen einer bestimmten Nukleobase auf dem Matrizenstrang erfolgt eine Elongation des Primers, ansonsten wird kein 5'-Aminonukleosidtriphosphat an den Primer angefügt. Der Reaktionsansatz wird anschließend mit einer 3'→5'-Exonuklease behandelt. Nach Anfügen des 5'-Amino-modifizierten Nukleotids an den Primer findet aufgrund der P-N-Bindung kein enzymatischer Abbau durch die Exonuklease statt und die im Primer eingebaute Markierungsgruppe bleibt am festen Träger immobilisiert. Wenn hingegen kein 5'-Amino-modifiziertes Nukleosidtriphosphat an den Primer angefügt wird, wird dieser durch die Exonuklease abgebaut und die Markierung vom festen Träger abgespalten. Das Verbleiben der Markierung am Träger oder deren Freisetzung kann ohne weiteres durch bekannte Methoden nachgewiesen werden.

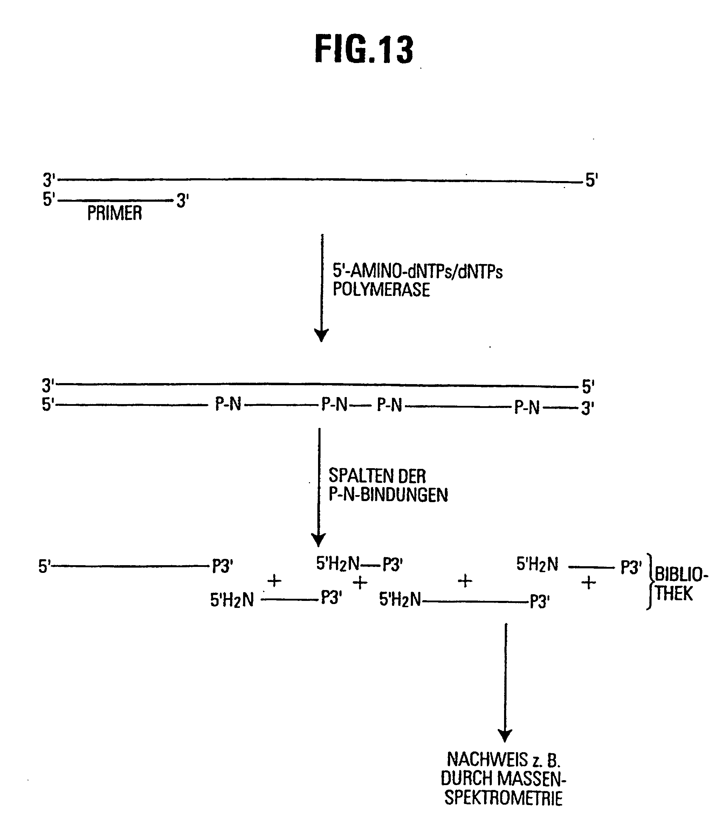

Fig. 13 zeigt die Erzeugung einer DNA-Bibliothek durch multiplen 5'-Aminodeoxynukleosidtriphosphat-Einbau in DNA Fragmente. Durch Spaltung der P-N-Bindungen wird eine Vielzahl unterschiedlicher DNA Fragmente erzeugt, die entweder an einen Träger gebunden werden können oder beispielsweise durch Massenspektrometrie analysiert werden können.

### Beispiele

### 1. Synthese von modifizierten Nukleosiden

Die Synthese modifizierter Nukleoside wurde in Anlehnung an Mag et al (25) durchgeführt. 5'-Amino-5'-deoxythymidin-5'-triphosphat und 5'-Amino-2',5'-dideoxycytidin-5'-triphosphat wurden gemäß Yamamoto et al (26) mittels Substitution der 5'-Hydroxylgruppe durch eine Azidgruppe gefolgt von katalytischer Reduktion zum entsprechenden Amin synthetisiert. Die entsprechenden Guanosin- und Adenosin-Derivate wurden durch Tosylierung der 5'-Hydroxylgruppe und Substitution der Tosylgruppe mit Lithiumazid hergestellt, um N¹-C⁵-Cyclisierungsreaktionen zu vermeiden. Die Purin-Azidverbindungen wurden ebenfalls durch katalytische Hydrierung reduziert. Nach Schutzgruppenabspaltung wurden die Nukleosidegemäßder von Letsinger et al (27) beschriebenen Methode mit Trinatriumtrimetaphosphat triphosphoryliert.

### 1.1 N⁴-Benzoyl-2'-deoxycytidin

1 Äquivalent 2'-Deoxycytidin wurde in wasserfreiem Pyridin gelöst. Hierzu wurden 5 Äquvalente Trimethylchlorsilan bei Raumtemperatur gegeben. Das Reaktionsgemisch wurde 30 min gerührt, auf 0°C abgekühlt und tropfenweise mit 1,2 Äquivalenten Benzoylchlorid versetzt. Das Gemisch wurde erst 30 min und dann weitere zwei Stunden bei Raumtemperatur gerührt. Die Reaktion wurde durch Zugabe von 10 ml kaltem Wasser bei 0°C gestoppt. Nach 20 min wurde das Lösungsmittel unter Vakuum entfernt. Der verbleibende Rückstand in heißem Wasser gelöst und dreimal mit Ethylacetat gewaschen. Die wässrige Phase wurde auf 4°C abgekühlt und die resultierenden Kristalle durch Filtration und Waschen mit kaltem Wasser gewonnen. Das Produkt wurde bei 50°C über P₂O₅ unter Vakuum auf konstantes Gewicht getrocknet.

N⁶-benxoyl-2'-deoxyadenosin und N²-Isobuturyl-2'-deoxyguanosin wurden nach der gleichen Vorschrift hergestellt.

### 1.2 5'-Azido-5'-deoxythymidin

7,26 g (30 mmol) Thymidin, 9,45 g (36 mmol) Triphenylphosphin, 5,85 g (90 mmol) Natriumazid und 11,94 g (36 mmol) Tetrabrommethan wurden in 120 ml trockenem Dimethylformamid gelöst und für 24 h bei Raumtemperatur gerührt. Das Gemisch wurde mit 150 ml NatriumhydrogencarbonatLösung gewaschen und die wässrige Phase wurde mit viermal 200 ml Chloroform extrahiert. Die organische Phase wurde mit Natriumsulfat getrocknet und das Lösungsmittel unter Vakuum entfernt. Das erhaltene Rohprodukt wurde durch Silicagelchromatographie mit einem Gradienten von 0 bis 10% Methanol in Dichlormethan gereinigt. Die Ausbeute war etwa 76% (6,09 g).

### Analyse:

- DC:: R_{f}: 0,40 (Chloroform: Methanol = 9:1; Silicagel 60, F₂₅₄, Merck)
- IR:: νₐₛ(N₃)=2093,7 cm⁻¹ (s, KBr)
NMR: δ-¹H[ppm], 270 MHz, 300 K, DMSO-d₆:
11,30 (s, 1 H, N³H); 7,47 (s, 1H, H⁶); 6,18 (t, 1H, H¹); 5,39 (d, 1H, O^{3'}H);
4,22 (m, 1H,H^{3'}); 3,85 (m, 1 H, H^{4'}); 3,55 (d, 2H, H^{5'1}, H^{5'2}); 2,29 (m, 1H, H^{2'2});
2,08 (m, 1 H, H^{2'1}); 1,79 (d, 3H, C⁵-CH₃)

### Elementaranalyse:

| | | | |
|---|---|---|---|
| berechnet | C: 44,94% | H: 4,90% | N: 26,21% |
| gefunden | C: 44,77% | H: 4,86% | N: 25,93% |

5'-Azido-N⁴-benzoyl-2',5'-dideoxycytidin wurde nach der gleichen Vorschrift hergestellt.

### 1.3 5'-Azido-2',5'-dideoxycytidin

5'-Azido-N⁴-benzoyl-2',5'-dideoxycytidin wurde in einer gesättigten Lösung von Ammoniak in Methanol gelöst und bei Raumtemperatur für 12 h gerührt. Dann wurde das Lösungsmittel im Vakuum abgezogen und der Rückstand chromatographisch unter Verwendung von Dichlormethan mit einem Gradienten von 0 bis 15% Methanol als Elutionsmittel gereinigt.

### 1.4 5'-O-(4-Methylbenzolsulfon)-N⁶-benzoyl-2'-deoxyadenosin und 5'-O-(4-Methylbenzolsulfon)-N²-isobutyryl-2'-deoxyguanosin

Jeweils 1 Äquivalent N⁶-Benzoyl-2'-deoxyadenosin und N²-Isobutyryl-2'deoxyguanosin wurden in trockenem Pyridin gelöst. Dazu wurden bei Raumtemperatur 3 Äquivalente 4-Methylbenzolsulfonylchlorid gegeben. Das Reaktionsgemisch wurde für 45 min gerührt, dann auf Eis gekühlt und mit 5 ml Wasser abgeschreckt. Nach 15 min wurde die Lösung eingedampft und der ölige Rückstand in Ethylacetat aufgenommen. Die Lösung wurde zweimal mit 5% NaHCO₃, Wasser und gesättigter Salzlösung gewaschen, über Natriumsulfat getrocknet und im Vakuum eingedampft. Der Rückstand wurde chromatographisch unter Verwendung eines Gradienten von 0 bis 10% Methanol in Dichlormethan als Elutionsmittel gereinigt.

### 1.5 5'-Azido-2',5'-dideoxyadenosin und 5'-Azido-2',5'-dideoxyguanosin

Jeweils 1 Äquivalent der in Beispiel 1.4 hergestellten Verbindungen wurde in trockenem N,N-Dimethylformamid aufgenommen und mit fünf Äquivalenten Lithiumazid versetzt. Die Lösung wurde für 5 h bei 50°C gerührt. Dann wurde das fünffache Volumen an Dichlormethan zugesetzt und dreimal mit Wasser gewaschen. Die organische Phase wurde mit Natriumsulfat getrocknet, abfiltriert und das Lösungsmittel abgezogen. Die Schutzgruppenabspaltung und Reinigung der Rohprodukte erfolgte wie bei der Herstellung von 5'-Azido 2',5'-dideoxycytidin beschrieben.

### 1.6. 5'-Amino 5'-deoxythymidin

150 ml absoluter Methanol wurden durch fünfmaliges Einfrieren und Auftauen unter Vakuum entgast. Darin wurden 1,5 g (5,62 mmol) 5'-Azido-5'-deoxythymidin gelöst und eine geringe Menge an Platindioxid-Hydrat-Katalysator zugesetzt. Wasserstoffgas wurde in die Lösung eingeleitet und das Gemisch wurde 2 h bei Raumtemperatur gerührt. Nach Filtration durch Cellite wurde das Lösungsmittel entfernt. Eine weitere Aufreinigung war nicht erforderlich. Die Ausbeute war nahezu quantitativ.

### Analyse:

- DC:: R_{f}: 0,05 (Dichlormethan:Methanol = 9:1; Silicagel 60 F₂₅₄, Merck)
- IR:: νₐₛ (N₃) = nicht vorhanden (KBr)
NMR: δ-¹H [ppm], 270 MHz, 300 K, DMSO-d₆:
7,63 (s, 1H, H⁶); 6,15 (t, 1H, H¹); 5,15 (d, 1H, O^{3'}H); 4,21 (m, 1 H, H^{3'});
3,65 (m, 1H, H^{4'}); 2,73 (d, 2H, H^{5'1}, H^{5'2}); 1,95-2,15 (m, 2H, H^{2'1}, H^{2'2}); 1,79 (d, 3H, C⁵-CH₃)

| | | | |
|---|---|---|---|
| Massenspektrometrie | ESI (+) | berechnet | 242,2 Da |
| | | gefunden | 242,1 Da |

5'-Amino-2',5'-dideoxycytidin, 5'-Amino-2',5'-dideoxyadenosin und 5'-Amino-2', 5'-dideoxyguanosin wurden nach der gleichen Vorschrift hergestellt.

### 1.7 5'-Amino-5'-deoxythymidin-5'-triphosphat

2 mg (1 Äquivalent) 5'-Amino-5'-deoxythymidin und 13,4 mg (5 Äquivalente) Trinatriumtrimetaphosphat wurden in 100 µl sterilem Wasser (pH 8,50) aufgelöst, 30 h bei Raumtemperatur gerührt und dann bei -80°C aufbewahrt. Für die Sequenzierungsexperimente wurde ein Aliquot direkt ohne weitere Reinigung aus dem Reaktionsgemisch entnommen.

5'-Amino-2',5'-dideoxycytidin-5'-triphosphat, 5'-Amino-2',5'-dideoxyadenosin-5'-triphosphat und 5'-Amino-2',5'-dideoxyguanoasin-5'-triphosphat wurden nach der gleichen Synthesevorschrift hergestellt.

### Analyse

Massenspektrometrie: ESI(-):
5'-Amino-2',5'-dideoxyadenosin-5'-triphosphat:
   - berechnet:: 490,199 Da
   - gefunden:: 488,8 Da (M-H);
   610,9 Da (M-2H+Na);
   532,7 Da (M-2H + 2Na)
5'-Amino-2',5'-dideoxycytidin-5'-triphosphat:
   - berechnet:: 466,173 Da
   - gefunden:: 464,8 Da (M-H);
   486,8 Da (M-2H + Na)
5'-Amino-2',5'-dideoxyguanosin-5'-triphosphat:
   - berechnet:: 506,198 Da
   - gefunden:: 504,8 Da (M-H);
   526,8 Da (M-2H+Na)
   548,7 Da (M-3H+2Na)
5'-Amino-5'-deoxythymidin-5'-triphosphat:
   - berechnet:: 481,184 Da
   - gefunden:: 479,9 Da (M-H);
   480,6 Da (M);
   501,8 Da (M-2H+Na)
   523,8 Da (M-3H + 2Na)

### 2. Sequenzierungsreaktion

Die Durchführung der Sequenzierungsreaktion ist am Beispiel der T-Spur dargestellt. Die Sequenzierungsreaktion für die A-, C- und G-Spuren kann auf analoge Weise erfolgen werden.

Pro Ansatz wurden 0,3 µl Primer, z.B. Universal- oder Reverseprimer (Fluoresceinisothiocyanat-markiert; 2 µM), 0,3 µl DMSO, 1,2 µl ss-M13 MP18 (+) DNA und 0,6 µl Annealingpuffer (1 M Tris-HCl pH 7, 6; 100 mM MgCl₂) zusammengegeben. Die Lösung wurde 3 min bei 70°C inkubiert und über eine Zeitdauer von 20 min auf Raumtemperatur abgekühlt.

Zu dieser Lösung wurden 0,60 µl einer dNTP-Mischung (jeweils 250 µM dATP, dCTP und dGTP sowie 50 µM dTTP), 0,44 µl 5'-Amino-dTTP (aus der Reaktionsmischung von Beispiel 1.7) und 0,25 µl T7 Polymerase (8 U/µl) zugegeben und 10 min bei 37°C inkubiert. Für die A-, C- und G-Spuren wurden entsprechend 5'-Amino-dATP, 5'-Amino-dCTPoder5'-Amino-dGTP verwendet.

Anschließend werden 4 µl Stoplösung (95% deionisiertes Formamid, 20 mM EDTA, 0,05% Xylencyanol; 0.05% Bromphenolblau) zugegeben.

Zur Herstellung eines sequenzierbaren Gemisches an Nukteinsäurefragmenten wurden die P-N-Bindungen gespalten. Hierzu stehen folgende alternative Methoden zur Verfügung:
1. Spaltung durch Temperaturerhöhung:
   40 minütiges Erwärmen auf 95°C (auch 20-minütiges Erwärmen ist möglich)
2. Spaltung unter sauren Bedingungen:
   Zugeben von 2 bis 5 µl 1 N HCl, fünfminütiges inkubieren bei Raumtemperatur, Neutralisieren mit 2 bis 5 µl 1 N NaOH, fünfminütiges Denaturieren bei 95°C
3. Spaltung durch Mikrowellen:
   Behandeln der Probe mit Mikrowellenstrahlung für 30 min bei 900 W.

Alternativ hierzu können auch enzymatische Spaltungsmethoden durch Exo- bzw. Endonukleasen, insbesondere durch 3'→5'-Exonukleasen wie etwa 3'→5'-Schlangengiftphosphodiesterase verwendet werden. Hierzu werden 10 mU (3,2 µl) 3'→5'Schlangengiftphosphodiesterase zugegeben, für 10 min 40°C inkubiert und für 5 min bei 95°C denaturiert.

Anschließend wurde das Fragmentgemisch analysiert, z.B. durch Polyacrylamidgelelektrophorese. Hierzu wurden 5 µl des Reaktionsansatzes auf das Gel geladen. Alternativ kann der Reaktionsansatz bei -20°C aufbewahrt und dann vor dem Aufbringen auf das Gel für 3 min bei 95°C denaturiert werden.

### Literaturverzeichnis:

(1) Sanger, F., Nicklen, S., Coulson, A.R., *Proc. Natl. Acad Sci*. USA 74, 5463-5467, 1977
(2) Pieles, U., Zürcher, W., Schär, M., Moser, H.E., *Nucl. Acids. Res*. 21, 3191-3196, 1993
(3) a) Jenes, D.H., *BioTechniques* 22, 938-946, 1997 b) Brenner, S., WO 95/27080, 1995
(4) Ronaghi, M., Kharamouhamed, S., Pettersson, B., Uhlén, M., Nyrén, P., *Anal. Biochem*. 242, 84-89, 1996
(5) Maxam, A.M., Gilbert, W., *Proc. Natl. Acad. Sci*. USA 74, 560-564, 1977
(6)
   a) Gish, G., Eckstein, *F., Science* 240, 1520-1522, 1988
   b) Nakayame, K.L., Gish, G., Eckstein, F., Vosberg, H.-P., *Nucl. Acids Res*. 16, 9947-9959, 1988
   c) Labeit, S., Lehrrach, H., Goody, R.S., *DNA* 5, 173-177, 1986
   d) Porter, K.W., Briley, J.D., Ramsay Shaw, B., *Nucl. Acids Res*. 25, 1611-1617, 1997
(7)
   a) Barnes, W.M., *J. Mol. Biol.* 119, 83-99, 1978
   b) Hamilton, R.T., Wu, R., *J. Biol. Chem.* 249, 2466-2472, 1974
(8)
   a) Canard, B., Sarfati, R.S., *Gene* 148, 1-6, 1994
   b) Carnard, B., Sarfati, S., PCT Int. Appl. WO 94 23064; PCT/FR94/00345, 1994
(9)
   a) Dramanc, R., Labat, I., Bruckner, I., Crkvenjakov, R, *Genomics* 4, 114-128, 1989
   b) Dramanc, R., Dramanc, S., Stroszka, Z., Paunesku, T., Labat, I., Zeremski, M., Snoddy, J., Funkhouser, W.K., Koop, B., Hood, L., Crkvenjakov, R., *Science* 260, 1649-1652, 1953
   c) Bains, W., Smith, G.C., *J. Theor. Biol.* 135, 303-307, 1988
(10)
   a) Baldwin, M.A., *Natural Products reports,* 33-44, 1995;
   b) Wolter, M.A., Engels, J.W., *Eur. Mass Spektrom.* 1, 583-590, 1995
   c) Nordhoff, E., Karas, M., Cramer, R., Hahner, S., Hillenkamp, F., Kirpekar, F., Lezius, A., Muth, J., Meier, C., Engeis, J.W., *J. Mass Spectrom.* 30, 99-112, 1995
   d) Little, D.P., Chorush, R.A., Speir. J.P., Senko, M.W., Kelleher, N.L., McLafferty, F.W., *J. Am. Chem. Soc.* 116, 4893-4897, 1994
   e) Grotjahn. L., Frank, R., Blöcker, H., *Nucl. Acids Res.* 10, 4671-4678, 1983
(11) Löber, G., Kittler, L., *PhiuZ* 27, 113-117, 1996
(12)
   a) Ansorge, W., Sproat, B., Stegemann, J., Schwager, C., *J. Biochem. Biophys. Meth.* 13, 315-323, 1986
   b) Smith, L.M., Sanders, J.Z., Kaiser, R.J., Hughes, P. Dodd, C., Conell, C.R., Heiner, C., Kent, S.B.H., Hood, L.E., *Nature* 321, 674-679, 1986
(13) Voss, H., Schwager, C., Wirkner, U., Zimmermann, J., Erfle, H., Hewitt, N.A., Rupp, T., Stegemann, J., Ansorge, W., *Meth. Mol. Cell. Biol.* 3, 30-34, 1992
(14) Lee, L.G., Connel, C.R., Woo, S.L., Cheng, R.D., McArdle, B.F., Fuller, C.W., Halloran, N.D., Wilson, R., *Nucl. Acids Res.* 20, 2471-2483, 1992
(15) Murray, V., *Nucl. Acids Res.* 17, 8889, 1989
(16) Rosenthal, A., Coutelle, O., Craxton, M., *Nucl. Acids Res.* 21, 173-174, 1993
(17) Amersham Liefe Science product catalogue 1996, 89
(18) Kilger, C., Pääbo, S., *Biol. Chem.* 378, 99-105, 1997
(19) Smith, L.M., Fung, S., Hunkapillar, T.J., Hood, L.E., *Nucl. Acids Res.* 13, 2399-2412, 1985
(20) Current protocols in Molecular Biology, Vol 1, Section 3.10, John Wiley and Sons, Series Editor: Virginia Benson Chanda
(21) Bruick, R.K., Koppitz, M., Joyce, G.F., Orgel L.E., *Nucl. Acids Res*. 25, 1309-1310, 1997
(22) Biomagnetic Techniques in Molecular Biology, Technical Handbook, second edition, Dynal, Oslo, Norway, 156-157
(23) Landegren, U., Laboratory protocols for mutation detection, Oxford University Press, 1996, ISBN 0-19-857795-8
(24) Nikiforov, T.T., Rendle, R., B., Goelet, B., Rogers, Y-H., Kotewicz, M., L., Anderson, S.; Trainor, G., L., Knapp, M.R., Genetic Bit Analysis: a solid phase method for typing single nucleotide polymorphisms, *Nucl., Acids Res.* 22, 4167-4175, 1994
(24a) Alexandrova, L.A., Skoblov, A.Y., Jasko, M.V., Victorova, L.S., Krayevsky, A.A., *Nucl. Acids Res.* 26, 778-786, 1998
(25) Mag, M., Engels, J.W., *Nucl. Acids Res.* 17, 5973-5988, 1989
(26) Yamamoto, J., Sekine, M., Hata, T., *J. Chem. Soc. Perkin Trans.* I 1, 306-310, 1980
(27) Letsinger, R.L., Wilkes, J.S., Dumas, L.B., *J. Am. Chem. Soc.,* 292-293, 1972
(28) J. Am. Chem. Soc. 94, 292 (1072)
(29) Nucleic Acids Res. 17, 5973 (1989)
(30) WO-A-95 06 752

## Patentansprüche

1. Verfahren zum Nachweis von Nukleinsäuren, umfassend das Einbauen von Verbindungen der allgemeinen Formel (I): 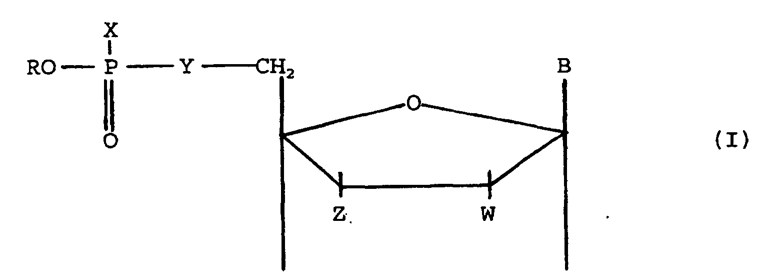 worin:
B eine Nukleobase bedeutet,
W und Z jeweils OR¹, SR¹, N(R¹)₂ oder R¹ bedeuten,
wobei R¹ jeweils unabhängig bei jedem Vorkommen Wasserstoff oder einen organischen Rest darstellt,
X OR², SR² oder B(R²)₃ bedeutet,
wobei R² jeweils unabhängig Wasserstoff, ein Kation oder einen organischen Rest bedeutet,
Y NR³ oder S bedeutet, wobei R³ Wasserstoff oder einen organischen Rest darstellt und
R Wasserstoff, ein Kation, einen organischen Rest oder eine gegebenenfalls modifizierte Phosphat- oder Diphosphatgruppe bedeutet,
in Nukleinsäuren und anschließende ortsspezifische Spaltung der Nukleinsäuren.

2. Verfahren nach Anspruch 1,
**dadurch gekennzeichnet,**
**dass** der Einbau enzymatisch erfolgt.

3. Verfahren nach Anspruch 2,
**dadurch gekennzeichnet,**
**dass** das Enzym ausgewählt wird aus der Gruppe bestehend aus DNA-abhängigen DNA Polymerasen, DNA-abhängigen RNA Polymerasen, RNA-abhängigen DNA Polymerasen, RNA-abhängigen RNA Polymerasen und Terminalen Transferasen.

4. Verfahren nach einem der Ansprüche 1 bis 3,
**dadurch gekennzeichnet,**
**dass** die ortsspezifische Spaltung erfolgt durch:
(i) Temperaturerhöhung,
(ii) Einstellen saurer Bedingungen,
(iii) Mikrowellenbehandlung,
(iv) Laserbehandlung oder/und
(v) enzymatischem Verdau.

5. Verfahren nach einem der Ansprüche 1 bis 4,
**dadurch gekennzeichnet,**
**dass** der Einbau der Verbindungen in Kombination mit einer Nukleinsäureamplifikationsreaktion erfolgt.

6. Verfahren nach Anspruch 5,
**dadurch gekennzeichnet,**
**dass** die Nukleinsäureamplifikation eine PCR umfasst.

7. Verfahren nach einem der Ansprüche 1 bis 6,
**dadurch gekennzeichnet,**
**dass** der Einbau der Verbindungen in trägergebundene Nukleinsäuren erfolgt.

8. Verfahren nach einem der Ansprüche 1 bis 7,
**dadurch gekennzeichnet,**
**dass** in die durch Spaltung entstehenden Nukleinsäurefragmente eine oder mehrere Markierungsgruppen eingebaut werden.

9. Verfahren nach Anspruch 8,
**dadurch gekennzeichnet,**
**dass** Markierungsgruppen am 5'- oder/und 3'-Ende der Nukleinsäurefragmente angefügt werden.

10. Verfahren nach einem der Ansprüche 1 bis 8,
**dadurch gekennzeichnet,**
**dass** die durch Spaltung entstehenden Nukleinsäurefragmente auf einem Träger immobilisiert werden.

11. Verfahren nach Anspruch 10,
**dadurch gekennzeichnet,**
**dass** der Träger eine Oberfläche aus Metall, Glas, Keramik oder/und Kunststoff aufweist.

12. Verfahren nach Anspruch 10 oder 11,
**dadurch gekennzeichnet,**
**dass** der Träger aus Mikropartikeln und Biochips ausgewählt wird.

13. Verfahren nach einem der vorhergehenden Ansprüche,
**dadurch gekennzeichnet,**
**dass** eine Nachweisreaktion an Nukleinsäuren, die in einer durch die Spaltung erzeugten Nukleinsäurebibliothek enthalten sind, durchgeführt wird.

14. Verfahren nach einem der Ansprüch 1 bis 13,
**dadurch gekennzeichnet,**
**dass** die Nachweisreaktion eine massenspektrometrische Analyse umfasst.

15. Verfahren nach einem der Ansprüche 1 bis 14,
**dadurch gekennzeichnet,**
**dass** die Nachweisreaktion eine Elektrophorese umfasst.

16. Verfahren nach einem der Ansprüche 1 bis 15,
**dadurch gekennzeichnet,**
**dass** die Nachweisreaktion eine Sequenzbestimmung umfasst.

17. Verfahren nach Anspruch 16,
**dadurch gekennzeichnet,**
**dass** die Sequenzbestimmung eine Zyklussequenzierung in Kombination mit einer Nukleinsäureamplifikation umfasst.

18. Verfahren nach Anspruch 16 oder 17,
**dadurch gekennzeichnet,**
**dass** die Sequenzbestimmung bidirektional auf einem Nukleinsäurestrang erfolgt.

19. Verfahren nach einem der vorhergehenden Ansprüche,
**dadurch gekennzeichnet,**
**dass** die durch Spaltung entstehenden Nukleinsäurefragmente für den Nachweis von Mutationen eingesetzt werden.

20. Verfahren nach einem der Ansprüche 1 bis 19,
**dadurch gekennzeichnet,**
**dass** die durch Spaltung entstehenden Nukleinsäurefragmente am 5'-Ende die Gruppe HY-CH₂- aufweisen, wobei Y wie in Anspruch 1 definiert ist.

21. Verfahren nach einem der Ansprüche 1 bis 20,
**dadurch gekennzeichnet,**
**dass** die Nukleinsäurefragmente am 3'-Ende eine Phosphatgruppe aufweisen.

22. Verwendung eines Reagenzienkits, der mindestens eine Verbindung der allgemeinen Formel (I) wie in Anspruch 1 definiert zusammen mit weiteren Nachweiskomponenten enthält, in einem Verfahren zum Nachweis von Nukleinsäuren nach einem der Ansprüche 1 bis 21.

## Claims

1. Method for detecting nucleic acids comprising the incorporation of compounds of the general formula (I): 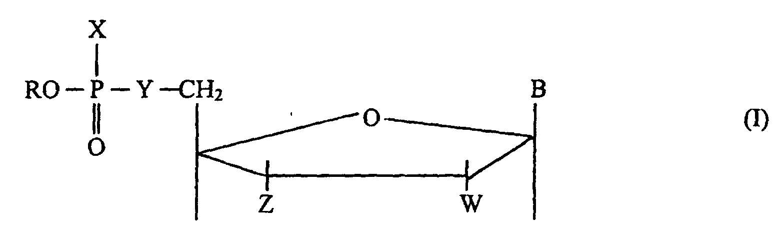 in which
B denotes a nucleobase,
W and Z each denote OR¹, SR¹, N(R¹)₂ or R¹ where R¹, independently at each occurrence represents hydrogen or an organic residue,
X denotes OR², SR² or B(R²)₃ where R² in each case independently denotes hydrogen, a cation or an organic residue,
Y denotes NR³ or S where R³ represents hydrogen or an organic residue and
R denotes hydrogen, a cation, an organic residue or an optionally modified phosphate or diphosphate group,
into nucleic acids and a subsequent site-specific cleavage of the nucleic acids.

2. Method as claimed in claim 1,
**characterized in that,**
the incorporation takes place enzymatically.

3. Method as claimed in claim 2,
**characterized in that**
the enzyme is selected from the group comprising DNA-dependent DNA polymerases, DNA-dependent RNA polymerases, RNA-dependent DNA polymerases, RNA-dependent RNA polymerases and terminal transferases.

4. Method as claimed in one of the claims 1 to 3,
**characterized in that**
the site specific cleavage is carried out by:
(i) increasing the temperature,
(ii) acidification,
(iii) microwave treatment,
(iv) laser treatment or/and
(v) enzymatic digestion.

5. Method as claimed in one of the claims 1 to 4,
**characterized in that**
the compounds are incorporated in combination with a nucleic acid amplification reaction.

6. Method as claimed in claim 5,
**characterized in that**
the nucleic acid amplification comprises a PCR.

7. Method as claimed in one of the claims 1 to 6,
**characterized in that**
the compounds are incorporated into support-bound nucleic acids.

8. Method as claimed in one of the claims 1 to 7,
**characterized in that**
one or more labelling groups are incorporated into the nucleic acid fragments that are formed by cleavage.

9. Method as claimed in claim 8,
**characterized in that**
the labelling groups are attached to the 5' or/and 3' end of the nucleic acid fragments.

10. Method as claimed in one of the claims 1 to 8,
**characterized in that**
the nucleic acid fragments formed by cleavage are immobilized on a support.

11. Method as claimed in claim 10,
**characterized in that**
the support has a surface made of metal, glas, ceramic or/and plastic.

12. Method as claimed in claim 10 or 11,
**characterized in that**
the support is selected from microparticles and biochips.

13. Method as claimed in one of the previous claims,
**characterized in that**
a detection reaction is carried out on nucleic acids which are contained in a nucleic acid library generated by the cleavage.

14. Method as claimed in one of the claims 1 to 13,
**characterized in that**
the detection reaction comprises a mass spectrometric analysis.

15. Method as claimed in one of the claims I to 14,
**characterized in that**
the detection reaction comprises an electrophoresis.

16. Method as claimed in one of the claims 1 to 15,
**characterized in that**
the detection reaction comprises a sequence determination.

17. Method as claimed in claim 16,
**characterized in that**
the sequence determination comprises a cyclic sequencing in combination with a nucleic acid amplification.

18. Method as claimed in claim 16 or 17,
**characterized in that**
the sequence determination is carried out bidirectionally on one nucleic acid strand.

19. Method as claimed in one of the previous claims,
**characterized in that**
the nucleic acid fragments formed by cleavage are used to detect mutations.

20. Method as claimed in one of the claims 1 to 19,
**characterized in that**
the nucleic acid fragments formed by cleavage have a HY-CH₂- group at the 5' end where Y is defined as in claim 1.

21. Method as claimed in one of the claims I to 20,
**characterized in that**
the nucleic acid fragments have a phosphate group at their 3' end.

22. Use of a reagent kit which contains at least one compound of the general formula (I) as defined in claim I together with other detection components in a method for detecting nucleic acids as claimed in one of the claims 1 to 21.

## Revendications

1. Procédé de caractérisation d'acides nucléiques, comprenant l'incorporation de composés de formule générale (I): 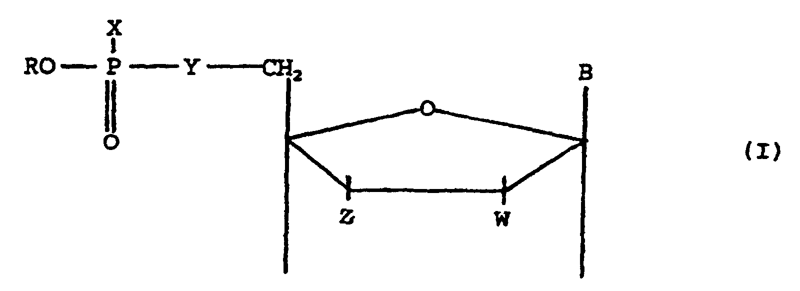 dans laquelle:
B représente une base nucléique,
W et Z représentent chacun OR¹, SR¹, N(R¹)₂ ou R¹, où R¹ représente indépendamment, à chaque fois qu'il apparaît, l'hydrogène ou un radical organique,
X représente OR², SR² ou B(R²)₃, où R² représente à chaque fois indépendamment l'hydrogène, un cation ou un radical organique,
Y représente NR³ ou S, où R³ représente l'hydrogène ou un radical organique, et
R représente l'hydrogène, un cation, un radical organique ou un groupement phosphate ou diphosphate éventuellement modifié,
dans des acides nucléiques, puis une coupure site-spécifique des acides nucléiques.

2. Procédé selon la revendication 1, **caractérisé en ce que** l'incorporation s'effectue par voie enzymatique.

3. Procédé selon la revendication 2, **caractérisé en ce que** l'enzyme est choisie dans le groupe constitué par des ADN-polymérases ADN-dépendantes, des ARN-polymérases ADN-dépendantes, des ADN-polymérases ARN-dépendantes, des ARN-polymérases ARN-dépendantes et des transférases terminales.

4. Procédé selon l'une des revendications 1 à 3, **caractérisé en ce que** la coupure site-spécifique s'effectue par
(i) élévation de la température,
(ii) établissement de conditions acides,
(iii) traitement aux micro-ondes,
(iv) traitement au laser et/ou
(v) digestion enzymatique.

5. Procédé selon l'une des revendications 1 à 4, **caractérisé en ce que** l'incorporation des composés s'effectue en combinaison avec une réaction d'amplification d'acide nucléique.

6. Procédé selon la revendication 5, **caractérisé en ce que** l'amplification d'acide nucléique comprend une PCR.

7. Procédé selon l'une des revendications 1 à 6, **caractérisé en ce que** l'incorporation des composés s'effectue dans des acides nucléiques liés à un support.

8. Procédé selon l'une des revendications 1 à 7, **caractérisé en ce que** l'on incorpore un ou plusieurs groupes marqueurs dans les fragments d'acides nucléiques formés par coupure.

9. Procédé selon la revendication 8, **caractérisé en ce que** l'on ajoute des groupes marqueurs à l'extrémité 5' et/ou 3' des fragments d'acides nucléiques.

10. Procédé selon l'une des revendications 1 à 8, **caractérisé en ce que** l'on immobilise sur un support les fragments d'acides nucléiques formés par coupure.

11. Procédé selon la revendication 10, **caractérisé en ce que** le support présente une surface de métal, de verre, de céramique et/ou de matière plastique.

12. Procédé selon la revendication 10 ou 11, **caractérisé en ce que** le support est choisi parmi des microparticules et des biopuces.

13. Procédé selon l'une des revendications précédentes, **caractérisé en ce que** l'on effectue une réaction de caractérisation sur des acides nucléiques qui sont contenus dans une banque d'acides nucléiques produite par la coupure.

14. Procédé selon l'une des revendications 1 à 13, **caractérisé en ce que** la réaction de caractérisation comprend une analyse par spectrométrie de masse.

15. Procédé selon l'une des revendications 1 à 14, **caractérisé en ce que** la réaction de caractérisation comprend une électrophorèse.

16. Procédé selon l'une des revendications 1 à 15, **caractérisé en ce que** la réaction de caractérisation comprend une détermination de la séquence.

17. Procédé selon la revendication 16, **caractérisé en ce que** la détermination de la séquence comprend un séquençage cyclique en combinaison avec une amplification d'acide nucléique.

18. Procédé selon la revendication 16 ou 17, **caractérisé en ce que** la détermination de la séquence s'effectue de façon bidirectionnelle sur un brin d'acide nucléique.

19. Procédé selon l'une des revendications précédentes, **caractérisé en ce que** l'on utilise les fragments d'acides nucléiques formés par coupure pour la mise en évidence de mutations.

20. Procédé selon l'une des revendications 1 à 19, **caractérisé en ce que** les fragments d'acides nucléiques formés par coupure présentent à l'extrémité 5' le groupement HY-CH₂- dans lequel Y a la définition donnée dans la revendication 1.

21. Procédé selon l'une des revendications 1 à 20, **caractérisé en ce que** les fragments d'acides nucléiques présentent un groupement phosphate à l'extrémité 3'.

22. Utilisation d'un kit de réactifs contenant au moins un composé de formule générale (I) tel que défini dans la revendication 1 avec d'autres constituants de caractérisation, dans un procédé de caractérisation d'acides nucléiques selon l'une des revendications 1 à 21.
